(19) Europäisches Patentamt
European Patent Office
Office européen des brevets

(11) **EP 2 345 733 A1**

(12) **EUROPEAN PATENT APPLICATION**
published in accordance with Art. 153(4) EPC

(43) Date of publication:
**20.07.2011 Bulletin 2011/29**

(51) Int Cl.:
***C12P 13/06*** $^{(2006.01)}$

(21) Application number: **09823674.8**

(86) International application number:
**PCT/JP2009/068611**

(22) Date of filing: **29.10.2009**

(87) International publication number:
**WO 2010/050564 (06.05.2010 Gazette 2010/18)**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**
Designated Extension States:
**AL BA RS**

(30) Priority: **31.10.2008 JP 2008281662**

(71) Applicant: **Kaneka Corporation**
**Osaka-shi, Osaka 530-8288 (JP)**
(72) Inventors:
• **HIRAI, Yoshinori**
**Takasago-shi**
**Hyogo 676-8688 (JP)**

• **MAEHARA, Katsuji**
**Osaka-shi**
**Osaka 530-8288 (JP)**
• **MOROSHIMA, Tadashi**
**Takasago-shi**
**Hyogo 676-8688 (JP)**
• **KANAMARU, Hiroyuki**
**Takasago-shi**
**Hyogo 676-8688 (JP)**
• **IWASAKI, Akira**
**Takasago-shi**
**Hyogo 676-8688 (JP)**

(74) Representative: **HOFFMANN EITLE**
**Patent- und Rechtsanwälte**
**Arabellastraße 4**
**81925 München (DE)**

(54) **PROCESS FOR PRODUCING N-PROTECTED AMINO ACID**

(57)     In the present invention, an N-protected amino acid is obtained by producing an amino acid using a biocatalyst such as a microorganism and enzyme; acidizing the reaction mixture containing the amino acid to adjust pH to not more than 4, without isolating the amino acid; and then protecting the amino group under a basic condition. As a result, a high-quality N-protected amino acid can be quantitatively and efficiently produced using a quantitative or slightly excessive protecting agent, even when the amino acid produced using a biocatalyst is directly protected without isolating the amino acid.

**EP 2 345 733 A1**

**Description**

TECHNICAL FIELD

The present invention relates to a process for producing an N-protected amino acid which is useful as an intermediate of a drug or an agricultural chemical.

BACKGROUND ART

An amino acid includes natural amino acids and nonnatural amino acid, both of which are very useful compounds and widely used as a raw material or an intermediate of a drug or an agricultural chemical.
When such an amino acid is used as a raw material or an intermediate compound, the carboxl group or/and amino group in the molecule thereof are protected before reaction to suppress the side reaction.
When an amino group is protected, in general, an N-protected amino acid can be rapidly and almost quantitatively obtained by using an equivalent amount or a little excessive amount of a protecting reagent in a basic condition (Non-patent Document 1 and Patent Document 1).

PRIOR ART

Patent Document

    Patent Document 1: JP2007-131589A

Non-patent Document

    Non-patent Document 1: "Jikken Kagaku Koza 5th edition (The Fifth Series of Experimental Chemistry)" edited by The Chemical Society of Japan, No.16, pp.221-226

DISCLOSURE OF THE INVENTION

PROBLEMS TO BE SOLVED BY THE INVENTION

An amino acid is often produced using a biocatalyst, since an optically active amino acid with high optical purity can be obtained. The amino acid production using a biocatalyst is generally carried out in an aqueous medium. When such a reaction is carried out in an aqueous medium, the isolation and purification of an amino acid are not efficient and difficult, since a troublesome procedure such as the separation of the amino acid having high water solubility and a water-soluble inorganic salt and the separation of the amino acid and fungus body or protein is needed. Needless to say, when the amino acid cannot be collected by said isolation and purification methods, the production cost becomes high. Therefore, in order to obtain a protected amino acid, it is preferable that the protection of an amino acid is conducted by directly-using the reaction mixture of producing the amino acid in terms or production efficiency and cost.
However, the present inventors found that in the case of using a biocatalyst in the production of an amino acid, an N-protected amino acid sometimes cannot be quantitatively produced by using a quantitative or slightly excessive protecting agent in the conventional methods. It was found that such a tendency especially becomes high when an amino acid is directly protected without isolating the obtained amino acid, and such a protecting reaction surprisingly becomes difficult to proceed even when an excessive protecting agent is used. By the above reasons, production efficiency is decreased and cost is increased in commercial large-scale production, and that makes difficult to provide a useful protected amino acid to market at low cost.
Under the above-mentioned situation, the objective of the present invention is to provide a process for efficiently producing an N-protected amino acid with high quality.

MEANS FOR SOLVING THE PROBLEMS

The present inventors studied very hard for solving the problems. As a result, the present inventors found that for effiently producing an N-protected amino acid from the amino acid produced using a biocatalyst, it is very effective to carry out an acidification treatment before the protecting reaction.

The present invention relates to a process for producing an N-protected amino acid, comprising the steps of producing an amino acid using a biocatalyst; acidizing the reaction mixture containing the amino acid to adjust pH to not more than 4, without isolating the amino acid; and then protecting the amino group under a basic condition.

EFFECT OF THE INVENTION

According to the present invention method, a high-quality N-protected amino acid can be quantitatively and efficiently produced using a quantitative or slightly excessive protecting agent, even when the amino acid produced using a biocatalyst is directly protected without isolating the amino acid.

MODE FOR CARRYING OUT THE INVENTION

First, the amino acid used in the present invention is described.

The amino acid used in the present invention is not particularly limited, and is specifically exemplified by glycine, alanine, 3-chloroalanine, β-alanine, valine, norvaline, leucine, norleucine, isoleucine, alloisoleucine, tert-leucine, phenylalanine, homophenylalanine, tyrosine, diiodotyrosine, threonine, allothreonine, serine, homoserine, isoserine, proline, hydroxyproline, 3,4-dehydroproline, tryptophan, thyroxine, methionine, homomethionine, cystine, homocystine, α-aminobutyric acid, β-aminobutyric acid, γ-aminobutyric acid, α-aminoisobutyric acid, aspartic acid, aspartic acid-β-cyclohexyl ester, aspartic acid-β-metyl ester, aspartic acid-β-isopropyl ester, aspartic acid-β-benzyl ester, glutamic acid, glutamic acid-γ-cyclohexyl ester, glutamic acid-γ-methyl ester, glutamic acid-γ-isopropyl ester, glutamic acid-γ-benzyl ester, lysine, hydroxylysine, ornithine, hydroxyornithine, arginine, histidine, anticapsin, adamantylglycine, taurine, γ-formyl-N-methylnorvaline, Ng-tosylarginine, O-benzylserine, O-benzylthreonine, $N^{in}$-formyltryptophan, 2-(2-amino-4-thiazolyl)-2-hydroxyiminoacetic acid, 2-(2-amino-4-thiazolyl)-2-methoxyiminoacetic acid, 2-(2-amino-4-thiazolyl)-2-glyoxyacetic acid, 2-(2-amino-4-thiazolyl)-2-pentanoic acid, 3-amino-2-hydroxy-4-phenylbutyric acid, 3-amino-3-phenylpropionic acid, phenylglycine, 4-hydroxyphenylglycine, 4-chlorophenylglycine, 4-chlorophenylalanine, cyclohexylalanine, cyclohexylglycine, 3-(1-naphthyl)alanine, 3-(2-naphthyl)alanine, creatine, azetidine-2-carboxylic acid, orcylalanine, ergothioneine, lanthionine, 1-methylhistidine, 3-methylhistidine and others.

Amino acids other than glycine have optical isomer and stereoisomer. In the present invention, any isomer may be used; and of course, the mixture of isomers and racemic form may also be used.

In addition, the carboxyl group of the amino acids may be transformed into other functional group. Specifically the transformed amino acid such as amide form and ester form may be preferably used.

The production of amino acid using a biocatalyst of the present invention may be carried out as the following methods; however, the present invention is not limited by the methods.

1) The corresponding keto acid is treated by an amino acid dehydrogenase or the culture of microorganism capable of producing the dehydrogenase in order to aminate the keto acid D-form-selectively or L-form-selectively. The amino acid dehydrogenase used in the reaction is exemplified by leucine dehydrogenase, alanine dehydrogenase, phenylalanine dehydrogenase, glutamic acid dehydrogenase, valine dehydrogenase, lysine dehydrogenase, aspartic acid dehydrogenase and others.

2) The corresponding keto acid is treated by an aminotransferase or the culture of microorganism capable of producing the aminotransferase in order to aminate the keto acid D-form-selectively or L-form-selectively.

3) DL-Amino acid alkyl ester is treated by an esterase or the culture of microorganism capable of producing the esterase in order to selectively hydrolyze the D-form or L-form thereof.

4) L-N-Acylamino acid is treated by an acylase or the culture of microorganism capable of producing the acylase in order to selectively hydrolyze the D-form or L-form thereof.

5) DL-Amino acid amide is treated by an amidase or the culture of microorganism capable of producing the amidase in order to selectively hydrolyze the D-form or L-form thereof.

6) 5-Substituted hydantoin is treated by a hydantoinase or the culture of microorganism capable of producing the hydantoinase or the processed material thereof in order to selectively hydrolyze the D-form or L-form thereof.

7) α-Aminonitrile compound is treated by a nitrilase or the culture of microorganism capable of producing the nitrilase in order to selectively hydrolyze the D-form or L-form thereof.

8) DL-Amino acid is treated by the culture of microorganism capable of selectively decomposing the D-form or L-form thereof in order to decompose either isomer of the amino acid.

9) DL-Amino acid is treated by a D- or L-amino acid oxidase, an amino acid dehydrogenase and an enzyme capable of regenerating a coenzyme or the culture of microorganism capable of producing the enzymes in order to transform the DL-amino acid into D-amino acid or L-amino acid in theoretical yield of 100%.

The term, "biocatalyst", of the present invention means an enzyme used for amino acid production, such as the above-described amino acid dehydrogenase, or a culture of a microorganism capable of producing the enzyme or a processed material thereof. The term, "culture of a microorganism" means a culture broth containing the microorganism or a cultured microorganism; and "processed material thereof" means a fractured microorganism obtained by fracturing the microorganism with a physics method or an enzyme, a crude extract obtained by removing insoluble material from the fractured microorganism with centrifugation and other method,
a freeze-dried microorganism, a microorganism dried by acetone, and others.

The "enzyme used for amino acid production" is exemplified by an amino acid dehydrogenase, an aminotransferase, an esterase, an acylase, an amidase, a hydantoinase, a nitrilase and others.

An amino acid dehydrogenase is exemplified by leucine dehydrogenase, alanine dehydrogenase, phenylalanine dehydrogenase, glutamic acid dehydrogenase, valine dehydrogenase, lysine dehydrogenase and aspartic acid dehydrogenase.

Specifically, leucine dehydrogenase and valine dehydrogenase are preferable as an amino acid dehydrogenase, in the case where the amino acid to be produced is an aliphatic amino acid such as valine, leucine, isoleucine, norvaline, norleucine and tert-leucine. When the amino acid to be produced is an aromatic amino acid such as phenylalanine, tyrosine, homophenylalanine and adamantylglycine, phenylalanine dehydrogenase is preferable as an amino acid dehydrogenase to be used. When the amino acid to be produced is glutamic acid or 6-hydroxynorleucine, glutamic acid dehydrogenase is preferable. When the amino acid to be produced is α-aminobutyric acid, α-aminovaleric acid, serine, glycine, 3-chloroalanine, 3-fluoroalanine or others, alanine dehydrogenase is preferable.

As leucine dehydrogenase, any enzyme having the activity of the dehydrogenase can be used; and an enzyme derived from a microorganism belonging to the genus Bacillus, the genus Thermoactinomyces, the genus Clostridium and the genus Corynebacterium are exemplified, preferably an enzyme derived from a microorganism such as Bacillus sphaericus, Bacillus stearothermophilus, Bacillus celeus, Bacillus subtilis, Thermoactinomyces intermedis and Clostridium thermoaceticum is exemplified, more preferably an enzyme derived from a microorganism such as Bacillus sphaericus NBRC3341 is exemplified.

As phenylalanine dehydrogenase, any enzyme having the activity of the dehydrogenase can be used; and an enzyme derived from a microorganism belonging to the genus Bacillus, the genus Sporosarcina, the genus Rhodococcus, the genus Thermoactinomyces, the genus Brevibacterium, the genus Nocardia and the genus Microbacterium is exemplified, preferably an enzyme derived from a microorganism such as Bacillus radius, Bacillus sphaericus, Sporosarcina ureae, Rhodococcus maris and Thermoactinomyces intermedius is exemplified, more preferably an enzyme derived from Bacillus badius IAM11059 is exemplified.

As the other dehydrogenase, any enzyme having a desired dehydrogenase activity can be used.

Each of the above microorganism can be obtained from patent microorganism authority depository and other research institute. For example, the microorganism identified by NBRC number can be obtained from Incorporated Administrative Agency National Institute of Technology and Evaluation Biological Resource Center, and the microorganism identified by IAM number can be obtained from Institute of Molecular and Cellular Biosciences of the University of Tokyo.

As the above-mentioned "microorganism capable of producing the enzyme for amino acid production", both of a wild strain and a mutant strain can be used. Alternatively the microorganism transformed by genetic method such as genetical modification can be also used.

The genetically-modified microorganism is exemplified by a transformed microorganism obtained by using a vector having a DNA which encodes an enzyme used for amino acid production.

In the case where an enzyme used for amino acid production is an amino acid dehydrogenase, the microorganism capable of producing both the dehydrogenase and an enzyme having the capability of regenerating a coenzyme on which the dehydrogenase depends, is preferably used. For example, a transformed microorganism obtained by using a vector having DNAs which encode an amino acid dehydrogenase and an enzyme having the capability of regenerating a coenzyme on which the dehydrogenase depends is exemplified, and preferably a transformed microorganism in which an enzyme having the capability of regenerating a coenzyme is formate dehydrogenase or glucose dehydrogenase is exemplified, and more preferably a transformed microorganism in which the formate dehydrogenase is derived from Thiobacillus sp. or the glucose dehydrogenase is derived from Bacillus megaterium, is exemplified. The host microorganism to be transformed by a vector is exemplified by a bacterium, a yeast, a filamentous fungus, a plant cell and an animal cell, and a bacterium is preferable in terms of easy transformation and expression efficiency of enzyme, and Escherichia coli is particularly preferable.

For example, when an enzyme for amino acid production is leucine dehydrogenase, a microorganism used as a biocatalyst is exemplified by a transformed microorganism obtained by using a vector having a DNA which encodes leucine dehydrogenase, and preferably a transformed microorganism obtained by using a vector having DNAs which encode leucine dehydrogenase and the above-mentioned formate dehydrogenase is exemplified. For example, Escherichia coli HB101 (pFTLB) which is described in WO2007/015511 and transformed using a vector having a DNA which encodes a enzyme derived from Bacillus sphaericus NBRC3341 as leucine dehydrogenase can be exemplified.

Keto acid or others can be treated with the above described enzyme or culture of a microorganism, for example, as follows. However, the treatment method is not limited by the following method.

To an appropriate solvent such as water, the corresponding keto acid, an inorganic salt containing formic acid and ammonia such as ammonium formate and ammonium sulfate, a coenzyme such as NAD$^+$, and a culture broth of the microorganism capable of producing the amino acid dehydrogenase and formate dehydrogenase or the cultured microorganism obtained from the culture broth are added. Subsequently the mixture is stirred for reaction under pH control.

The temperature of the reaction is 10 to 70°C, preferably 20 to 60°C; and the pH of the reaction mixture is maintained between 4 and 12, preferably between 6 and 10. Keto acid is preferably added so that the concentration thereof becomes 0.1 w/w% to 60 w/w%, preferably 1 w/w% to 30 w/w%. Keto acid may be added all at once or separately.

The reaction product containing the amino acid produced by the above-mentioned method is generally obtained as an aqueous solution. The solution may contain organic solvent and further inorganic salt. In the present invention method, protecting reaction may be carried out in the solution or slurry containing amino acid without isolating the amino acid. Alternatively the procedure for purification, such as filtration to remove insoluble matter, may be carried out before protecting reaction. The possible procedure is specifically exemplified by filtration of insoluble matter such as protein, removing solvent, treatment using activated carbon, separation of microorganism by centrifugal sedimentation, removing inorganic salts using ion-exchange resin and others. The procedure is not necessarily carried out before acidification treatment, and may be preferably carried out after acidification treatment. In addition, two or more procedures may be possibly combined.

Next, acidification treatment is described. By the treatment, the production efficiency of N-protected amino acid can be improved.

The acid used for the acidification treatment is not particularly limited, and for example, a mineral acid such as hydrochloric acid, sulfuric acid and nitric acid, and an organic acid such as methanesulfonic acid and ethanesulfonic acid are generally used. One of the acids may be used by itself, or plural acids may be combined to be used.

The form of acid for addition is not limited, and only acid may be added or the acid diluted with water and/or organic solvent may be used. When the acid is diluted with organic solvent to be used, the organic solvent is not limited as long as the solvent is not reacted with acid. Specifically methanol, ethanol, 2-propanol and toluene and the like are exemplified as usable organic solvent.

It is preferable that the acid is mixed with stirring so that the mixture does not locally become strongly acidic.

In the present invention, the pH of the solution is adjusted to not more than 4, preferably not more than 3, more preferably not more than 2, by the addition of the acid. As a result, N-protected amino acid can be efficiently obtained even when a protecting agent is used in a theoretic equivalent amount or slightly excessive amount.

The time for maintaining the pH of the solution is preferably not less than 5 minutes, more preferably not less than 30 minutes. Needless to say, production efficiency is decreased when the pH of the solution is maintained for a long time, and the optimum time for maintaining the pH may be decided by a preliminary experiment.

The temperature of the above procedures is not limited, but the temperature is generally set in the range of 0 to 50°C for easier operation.

The amino acid processed by the above-described acidification treatment can be rapidly and almost quantitatively protected, even when a protecting agent is used in a theoretic equivalent amount or slightly excessive amount.

The protecting agent used for protecting amino group is not particularly limited, and conventional agent may be used. For example, an N-alkoxycarbonylating agent, an N-carbamoylating agent and an N-acylating agent may be used. More specifically an alkyl haloformate, a dicarbonic acid dialkyl ester, an isocyanatoalkyl ester, a carbonic anhydride and an alkylcarbonyl halide are preferably used. Among the examples, methyl chloroformate, ethyl chloroformate, benzyl chloroformate, dicarbonic acid dimethyl ester, dicarbonic acid diethyl ester, dicarbonic acid di-tert-butyl ester, isocyanato-tert-butyl ester, isocyanatoisopropyl ester, isocyanatophenyl ester, benzoyl chloride, acetyl chlorine and acetic anhydride are particularly preferably used.

The conventional condition for the protecting reaction may be applied. The optimum condition cannot be completely decided, since the optimum condition is varied depending on the combination of amino acid and protecting agent to be used. In general, the reaction is carried out under basic a condition, and the pH for the reaction is 8 to 14, preferably 9 to 14; and the reaction temperature is -5 to 90°C, preferably 0 to 50°C.

The amount of the protecting agent is generally not less than 0.95 times by mole and 1.20 times by mole, preferably 0.98 times by mole and 1.10 times by mole, more preferably 0.99 times by more and 1.05 times by mole, relative to the amino acid.

By the above described method, N-protected amino acid can be efficiently produced from the amino acid produced using a biocatalyst.

The N-protected amino acid produced by the above described protecting reaction can be subsequently purified and isolated with a general purification method such as crystallization, fractional distillation and column chromatography.

EXAMPLES

The present invention is hereinafter explained in more detail with examples; however, the present invention is not limited by the examples.

Reference Example 1: Production of L-tert-leucine using cultured microorganism having leucine dehydrogenase activity

The 2xYT culture broth consisting of 1.6 w/v% Bacto Trypton, 1.0 w/v% Bacto Yeast Extract and 0.5 w/v% NaCl (pH7, 50 ml) was dispensed into 500 ml Sakaguchi flask, and steam pasteurization was carried out at 120°C for 20 minutes.

The transformed Escherichia coli HB101 (pFTLB) which was obtained by the method described in WO2007/015511 and had leucine dehydrogenase activity and formate dehydrogenase activity, was inoculated, and aerobically cultured with shaking at 37°C for 30 hours, to obtain a culture broth of the microorganism having leucine dehydrogenase activity and formate dehydrogenase activity. The microorganism was collected from the culture broth by centrifugation, and dispersed in the supernatant of the culture broth such that the concentration of the microorganism became 20 times compared to the culture broth. Into a 1 L volume separable flask, 3,3-dimethyl-2-oxo-butanoic acid (DMOB, 40 g) was added, and the pH thereof was adjusted to 7.3 with a 30 wt% sodium hydroxide aqueous solution. Subsequently ammonium formate (19.4 g), ammonium sulfate (8.2 g), Zinc sulfate heptahydrate (40 mg), NAD (61 mg), ion-exchanged water (256 g) and the microorganism suspension having formate dehydrogenase activity of 3200 u were added thereto. The mixture was stirred at 33°C for 19 hour for reaction, while the pH was controlled to 7.3 using 55 wt% sulfuric acid. The optical purity and content amount of generated L-tert-leucine were analyzed using high-performance liquid chromatography (HPLC); as a result, it was found that the microorganism reaction mixture containing 38.8 g of L-tert-leucine having an optical purity of 100%e.e. was obtained. Further, the microorganism was removed from the part of the obtained microorganism reaction mixture by centrifugation, to obtain the aqueous solution containing L-tert-leucine. Just for information, the content amount of protein was 0.0 to 1.1 mg/L.

In said HPLC analysis, SUMICHIRAL OA-5000 (manufactured by Sumika Chemical Analysis Service, Ltd., 4.6 mum × 150 mm) was used as a column, a mixed solution containing 2 mM copper sulfate aqueous solution and methanol at the volume ratio of 95 : 5 was used as a moving phase, flow speed was set at 1.0 mL/min, column temperature was set at 40°C, and detection wavelength was set at 210 nm.

Example 1

The pH of the microorganism reaction mixture which was obtained in accordance with Reference Example and contained L-tert-leucine (2.48 g, 18.4 mmol) was adjusted to 4.0 using concentrated hydrochloric acid. After the mixture was stirred for 2 hours, the pH thereof was adjusted 7.0 using a 30 wt% sodium hydroxide aqueous solution, and then the microorganism was separated by centrifugation.

Next the pH of thus obtained supernatant was adjusted to 10.6 using a 30 wt% sodium hydroxide aqueous solution. Subsequently the mixture was concentrated under reduced pressure while the temperature was kept at not more than 40°C, such that the solution amount became 30.1 g.

To the solution cooled with ice, methyl chloroformate (1.78 g, 18.8 mmol), 1.02 times by mole) was slowly added with stirring. The pH was lowered as the addition of ethyl chloroformate; however the pH was maintained between 9.5 and 10.5 by adding a 30 wt% sodium hydroxide aqueous solution at the same time. After the addition of methyl chloroformate, the mixture was stirred for 1 hour. Then HPLC analysis was carried out; as a result, it was found that the conversion ratio was 98%.

The conversion ratio was calculated using the following calculating formula.

```
Conversion ratio (%) = [(the peak dimension value of the
generated N-protected amino acid) / (the peak dimension value
```

of the amino acid + the peak dimension value of the generated N-protected amino acid)] × 100

In said HPLC analysis, CAPCELLPAKSCX (250 mm X 4.6 mm i.d.) was used as a column, a mixed solution containing a phosphate buffer (pH = 3.3) and acetonitrile at the volume ratio of 95 : 5 was used as a moving phase, flow speed was set at 1.0 mL/min, column temperature was set at 35°C, and a differential refractive index meter was used as a detector.

The same calculation was carried out in the following Examples.

Example 2

The pH of the aqueous solution containing L-tert-leucine (3.01 g, 23.0mmol) obtained in accordance with Reference Example was adjusted to 2.0 using concentrated hydrochloric acid, and then the mixture was allowed to stand for 2 hours. Next, the pH thereof was adjusted to 10.6 using a 30 wt% sodium hydroxide aqueous solution. The mixture was subsequently concentrated under reduced pressure while the temperature was kept at 40°C or less in order to decrease the solution amount to 29.7 g.

To the solution cooled with ice, methyl chloroformate (2.19 g, 23.1 mmol, 1.01 times by mole) was slowly added with stirring. The pH was lowered as the addition of methyl chloroformate; however the pH was maintained between 9.8 and 12.9 by adding a 30 wt% sodium hydroxide aqueous solution at the same time. After the addition of methyl chloroformate, the mixture was stirred for 2 hours. Then HPLC analysis was carried out; as a result, it was found that the conversion ratio was 99%.

Example 3

The pH of the microorganism reaction mixture which was obtained in accordance with Reference Example and contained L-tert-leucine (1.96 g, 14.9 mmol) was adjusted to 4.0 using concentrated hydrochloric acid. After the mixture was stirred for 1 hour, the microorganism was separated by centrifugal sedimentation.

Next the pH of thus obtained supernatant was adjusted to 10.6 using a 30 wt% sodium hydroxide aqueous solution. Subsequently the mixture was concentrated under reduced pressure while the temperature was kept at 40°C or less, such that the solution amount became 20.1 g.

To the solution cooled with ice, methyl chloroformate (1.63 g, 15.0 mmol), 1.01 times by mole) was slowly added with stirring. The pH was lowered as the addition of methyl chloroformate; however the pH was maintained between 8.9 and 12.8 by adding a 30 wt% sodium hydroxide aqueous solution at the same time. After the addition of methyl chloroformate, the mixture was stirred for 2 hours. Then HPLC analysis was carried out; as a result, it was found that the conversion ratio was 96%.

Example 4

The pH of the aqueous solution which was obtained in accordance with Reference Example and contained L-tert-leucine (2.05 g, 15.6 mmol) was adjusted to 2.0 using concentrated sulfuric acid, and then the mixture was allowed to still stand for 30 minutes. Next, after the pH of the mixture was adjusted to 10.7 using a 30 wt% sodium hydroxide aqueous solution, the mixture was concentrated under reduced pressure while the temperature was kept at 40°C or less, such that the solution amount became 19.8 g.

To the stirred solution, benzyl chloroformate (2.70 g, 15.8 mmol, 1.00 time by mole) was slowly added while the temperature was kept at 25°C or less. The pH was lowered as the addition of benzyl chloroformate; however the pH was maintained between 9.9 and 12.5 by adding a 30 wt% sodium hydroxide aqueous solution at the same time. After the addition of benzyl chloroformate, the mixture was stirred for 2 hours. Then HPLC analysis was carried out; as a result, it was found that the conversion ratio was 97%.

Example 5

The pH of the aqueous solution which was obtained in accordance with Reference Example and contained L-tert-leucine (2.03 g, 15.5 mmol) was adjusted to 2.0 using concentrated hydrochloric acid, and then the mixture was allowed to still stand for 30 minutes. Next the pH thereof was adjusted 10.7 using a 30 wt% sodium hydroxide aqueous solution, and then the mixture was concentrated under reduced pressure while the temperature was kept at not more than 40°C, such that the solution amount became 19.6 g.

To the stirred solution, dicarbonic acid di-tert-butyl ester (3.41 g, 15.6 mmol), 1.00 time by mole) was slowly added at room temperature. The pH was lowered as the addition of dicarbonic acid di-tert-butyl ester; however the pH was maintained between 9.5 and 10.5 by adding a 30 wt% sodium hydroxide aqueous solution at the same time. After the addition of dicarbonic acid di-tert-butyl ester, the mixture was stirred for 2.5 hours. Then HPLC analysis was carried out; as a result, it was found that the conversion ratio was 99%.

Comparative Example 1

The pH of the aqueous solution which was obtained in accordance with Reference Example and contained L-tert-leucine (3.01 g, 23.0 mmol) was adjusted to 10.6 using a 30 wt% sodium hydroxide aqueous solution, and then the mixture was concentrated under reduced pressure while the temperature was kept at 40°C or less, such that the solution amount became 30.3 g.

To the solution cooled with ice, methyl chloroformate (2.20 g, 23.3 mmol, 1.01 times by mole) was slowly added with stirring. The pH was lowered as the addition of methyl chloroformate; however the pH was maintained between 10.0 and 13.0 by adding a 30 wt% sodium hydroxide aqueous solution at the same time. After the addition of methyl chloroformate, the mixture was stirred for 2 hours. Then HPLC analysis was carried out; as a result, it was found that the conversion ratio was 86%.

Comparative Example 2

The pH of the aqueous solution which was obtained in accordance with Reference Example and contained L-tert-leucine (3.01 g, 23.0 mmol) was adjusted to 10.6 using a 30 wt% sodium hydroxide aqueous solution, and then the mixture was concentrated under reduced pressure while the temperature was kept at 40°C or less, such that the solution amount became 30.3 g.

To the solution cooled with ice, methyl chloroformate (2.20 g, 23.3 mmol), 1.01 times by mole) was slowly added with stirring. The pH was lowered as the addition of methyl chloroformate; however the pH was maintained between 10.0 and 13.0 by adding a 30 wt% sodium hydroxide aqueous solution at the same time. After the addition of methyl chloroformate, the mixture was stirred for 2 hours. Then HPLC analysis was carried out; as a result, it was found that the conversion ratio was 86%.

To the solution, methyl chloroformate (0.59 g, total use amount: 2.79 g, total 29.5 mmol) and 1.28 times by mole) was further added, while the pH of the reaction mixture was maintained between 10.0 and 13.0 by adding a 30 wt% sodium hydroxide aqueous solution at the same time. After the addition, the mixture was stirred for 2 hours. Then HPLC analysis was carried out; as a result, it was found that the conversion ratio was 90%.

Methyl chloroformate (1.28 g, total use amount: 4.07 g, total 43.1 mmol and 1. 87 times by mole) was further added in the same manner. After the addition, the mixture was stirred for 2 hours. Then HPLC analysis was carried out; as a result, it was found that the conversion ratio was 94%.

The result shows that protecting reaction does not sufficiently proceed in the case where acidification treatment is not carried out even when excessive protecting agent is used.

Comparative Example 3

The pH of the aqueous solution which was obtained in accordance with Reference Example and contained L-tert-leucine (2.05 g, 15.6 mmol) was adjusted to 5.1 using concentrated sulfuric acid, and then the mixture was allowed to stand for 30 minutes. Next the pH thereof was adjusted to 10.6 using a 30 wt% sodium hydroxide aqueous solution, and then the mixture was concentrated under reduced pressure while the temperature was kept at 40°C or less, such that the solution amount became 19.5 g.

To the solution cooled with ice, methyl chloroformate (1.49 g, 15.8 mmol, 1.01 times by mole) was slowly added with stirring. The pH was lowered as the addition of methyl chloroformate; however the pH was maintained between 9.9 and 11.4 by adding a 30 wt% sodium hydroxide aqueous solution at the same time. After the addition of methyl chloroformate, the mixture was stirred for 2 hours. Then HPLC analysis was carried out; as a result, it was found that the conversion ratio was 89%.

Comparative Example 4

The pH of the aqueous solution which was obtained in accordance with Reference Example and contained L-tert-leucine (1.52 g, 11.6 mmol) was adjusted to 30.6 using a 30 wt% sodium hydroxide aqueous solution, and then the mixture was concentrated under reduced pressure while the temperature was kept at 40°C or less, such that the solution amount became 15.7 g.

To the stirred solution, dicarbonic acid di-tert-butyl ester (2.63 g, 12.1 mmol, 1.04 times by mole) was slowly added. The pH was lowered as the addition of dicarbonic acid di-tert-butyl ester; however the pH was maintained between 9.6 and 10.5 by adding a 30 wt% sodium hydroxide aqueous solution at the same time. After the addition of dicarbonic acid di-tert-butyl ester, the mixture was stirred for 2.5 hours. Then HPLC analysis was carried out; as a result, it was found that the conversion ratio was 92%.

**Claims**

1. A process for producing an N-protected amino acid, comprising the steps of
   producing an amino acid using a biocatalyst;
   acidizing the reaction mixture containing the amino acid to adjust pH to not more than 4, without isolating the amino acid; and then
   protecting the amino group under a basic condition.

2. The production process of an N-protected amino acid according to claim 1, wherein the amino acid is tert-leucine.

3. The production process of an N-protected amino acid according to claim 1 or 2, wherein a protecting agent for protecting the amino group is an N-alkoxycarbonylating agent, an N-oarbamoylating agent or an N-acylating agent.

4. The production process of an N-protected amino acid according to claim 3, wherein a protecting agent for protecting the amino group is methyl chloroformate, ethyl chloroformate, benzyl chloroformate or dicarbonic acid di-tert-butyl ester.

5. The production process of an N-protected amino acid according to any one of claims 1 to 4, wherein the biocatalyst is an amino acid dehydrogenase, an aminotransferase, an esterase, an acylase, an amidase, a hydantoinase, a nitrilase, or a culture of a microorganism capable of producing the enzyme.

6. The production process of an N-protected amino acid according to claim 5, wherein the biocatalyst is an amino acid dehydrogenase or a culture of a microorganism capable of producing the enzyme.

7. The production process of an N-protected amino acid according to claim 6, wherein the amino acid dehydrogenase is leucine dehydrogenase, alanine dehydrogenase, phenylalanine dehydrogenase, glutamic acid dehydrogenase, valine dehydrogenase, lysine dehydrogenase or asparagine acid dehydrogenase.

8. The production process of an N-protected amino acid according to claim 7, wherein the amino acid dehydrogenase is derived from Bacillus sphaericus.

## INTERNATIONAL SEARCH REPORT

| International application No. |
| --- |
| PCT/JP2009/068611 |

**A. CLASSIFICATION OF SUBJECT MATTER**
*C12P13/06*(2006.01)i

According to International Patent Classification (IPC) or to both national classification and IPC

**B. FIELDS SEARCHED**

Minimum documentation searched (classification system followed by classification symbols)
C12P13/06

Documentation searched other than minimum documentation to the extent that such documents are included in the fields searched

| | | | |
| --- | --- | --- | --- |
| Jitsuyo Shinan Koho | 1922-1996 | Jitsuyo Shinan Toroku Koho | 1996-2009 |
| Kokai Jitsuyo Shinan Koho | 1971-2009 | Toroku Jitsuyo Shinan Koho | 1994-2009 |

Electronic data base consulted during the international search (name of data base and, where practicable, search terms used)
CA/BIOSIS/MEDLINE/WPIDS(STN), PubMed, JSTPlus(JDreamII),
JMEDPlus(JDreamII), JST7580(JDreamII)

**C. DOCUMENTS CONSIDERED TO BE RELEVANT**

| Category* | Citation of document, with indication, where appropriate, of the relevant passages | Relevant to claim No. |
| --- | --- | --- |
| Y | JP 2004-175703 A (Mitsubishi Rayon Co., Ltd.), 24 June 2004 (24.06.2004), (Family: none) | 1-8 |
| Y | JP 2007-131589 A (Mitsubishi Rayon Co., Ltd.), 31 May 2007 (31.05.2007), (Family: none) | 1-8 |
| Y | WO 2007/015511 A1 (Kaneka Corp.), 08 February 2007 (08.02.2007), & EP 1918375 A1 | 1-8 |
| Y | JP 40-6204 B1 (Ajinomoto Co., Inc.), 27 March 1965 (27.03.1965), (Family: none) | 1-8 |

☒ Further documents are listed in the continuation of Box C. ☐ See patent family annex.

| | |
| --- | --- |
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search<br>16 November, 2009 (16.11.09) | Date of mailing of the international search report<br>01 December, 2009 (01.12.09) |
| --- | --- |
| Name and mailing address of the ISA/<br>Japanese Patent Office | Authorized officer |
| Facsimile No. | Telephone No. |

Form PCT/ISA/210 (second sheet) (April 2007)

**INTERNATIONAL SEARCH REPORT**

| International application No. |
| --- |
| PCT/JP2009/068611 |

C (Continuation).   DOCUMENTS CONSIDERED TO BE RELEVANT

| Category* | Citation of document, with indication, where appropriate, of the relevant passages | Relevant to claim No. |
| --- | --- | --- |
| Y | US 3205261 A  (Kyowa Hakko Kogyo Co., Ltd.),<br>07 September 1965 (07.09.1965),<br>& GB 973828 A          & DE 1211118 B<br>& FR 1307136 A          & CH 415665 A<br>& NL 272094 A          & NL 122837 C<br>& BR 6243453 D          & AT 250882 B | 1-8 |
| Y | US 3147302 A  (Kyowa Hakko Kogyo Co., Ltd.),<br>01 September 1964 (01.09.1964),<br>& GB 937058 A          & DE 1417592 A<br>& NL 121837 C          & NL 268327 A | 1-8 |
| Y | Edited by Koichi YAMADA, Amino-san Hakko (Ue)<br>Soron, Kyoritsu Shuppan Co., Ltd., 20 February<br>1972 (20.02.1972), page 342 | 1-8 |

Form PCT/ISA/210 (continuation of second sheet) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2007131589 A

- WO 2007015511 A

**Non-patent literature cited in the description**

- The Fifth Series of Experimental Chemistry. Jikken Kagaku Koza. 221-226